**Europäisches Patentamt**

**European Patent Office**

**Office européen des brevets**

(19)

(11) Publication number: **0 201 134**
A2

## (12) EUROPEAN PATENT APPLICATION

(21) Application number: **86200767.1**

(22) Date of filing: **02.05.86**

(51) Int. Cl.⁴: **A 61 K 7/32**

(30) Priority: **08.05.85 IT 2061885**

(43) Date of publication of application: **12.11.86**
**Bulletin 86/46**

(84) Designated Contracting States: **AT BE CH DE FR GB IT LI LU NL SE**

(71) Applicant: **EURAND ITALIA S.p.A., Via Privata Pasteur 1, I-20092 Cinisello Balsamo, Milano (IT)**

(72) Inventor: **Gentilini, Leonardo, Via Alessandro Paoli, 4, I-20124 Milano (IT)**

(74) Representative: **Riccardi, Sergio, Riccardi & Co. Via Macedonio Melloni, 32, I-20129 Milano (IT)**

(54) Deodorant formulation with modulating effect on perspiration.

(57) A deodorant formulation with a modulating effect on perspiration is disclosed, using microcapsules of tannic acid and/or derivatives thereof as active astringent and deodorant agents, with the preferable addition of perfume and anti-bacterial agent, in such a way so as to obtain an effective sustained action in time, and modulated in relation to the extent of the perspiration of the person.

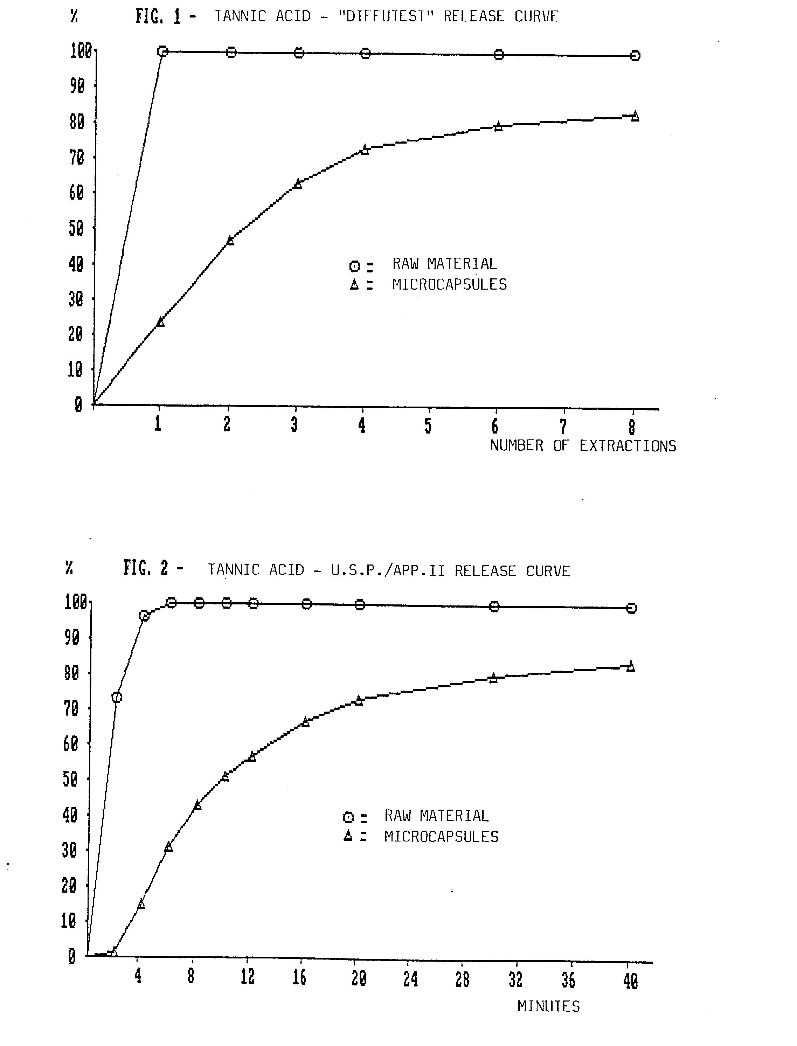

FIG. 1 - TANNIC ACID - "DIFFUTEST" RELEASE CURVE

O = RAW MATERIAL
Δ = MICROCAPSULES

NUMBER OF EXTRACTIONS

EP 0 201 134 A2

## DEODORANT FORMULATION WITH MODULATING EFFECT ON PERSPIRATION

The present invention relates to the use of tannic acid microcapsules and/or microcapsules of a perfume for the embodiment of an anti-perspirant deodorant of a new type, wherein the accumulation of the sustained effect of the microcapsules themselves and the modulation of the anti-perspirant activity function in relation to the extent of perspiration is achieved.

The formation of bad body-odour is correlated with sweat secreted by the apocrine glands which are essentially confined to the arm-pit, pectoral, anal and genital zones. In fact, it is the sweat secreted by these glands which is composed, in high percentage, of substances such as proteins, lipoproteins and lipids, the degradation of which, due to the micro-organisms normally present on the skin and in the sebaceous glands, is the cause of unpleasant odours.

Two approaches have been suggested for the control of body odour, namely:
- local application of suitable antiseptics which allay or hinder the decomposition of the products, especially the fats present at skin level, by bacteria, namely, the use of deodorants;
- local application of suitable astringent substances to reduce the intensity of perspiration, namely, the use of anti-perspirants.

The most modern orientation is that of formulating preparations wherein the two activities are synergically exploited, using both deodorant substances and anti-perspirants. The addition of a perfume is generally done so as to render these formulations more pleasant when in use.

Generally, however, these formulations show certain drawbacks, precisely:

- they have a violent impact and, with regard to the perfume, a relatively brief duration;

-they react independently to the degree of perspiration;

- the products commonly used as astringents, mostly aluminium salts and sometimes zirconium salts, have an irritating action on sensitive skins causing unpleasant sensations, and moreover, the metal salts used, often interfere with the perfumes, reducing their stability and modifying their olfactory properties;

- the perfumes, not suitably protected, modify themselves in time, changing the organolectic characteristics and become, in extreme cases, even unpleasant.

In the present invention, these serious drawbacks which are the main cause of limited use of these types of products on certain markets, have been overcome in the manner which is now described.

Tannic acid was used as astringent agent. It is a natural substance which is found in fruits and in the bark of several plants, but most of all in chestnut and quebracho wood and in the acorns of oaks, wherefrom it is extracted.

The astringent effect is well known and has been exploited for a long time in the pharmaceutical field, both for internal and external use (Martindale: The Extra Pharmacopoeia, Ed. 28, pag. 287).

Besides the specific astringent activity, tannic acid has a deodorant effect in that it inhibits the bacterial activity through the denaturation of the protreinic substances. Its intrinsic acidity also plays an important role: as can be seen in Table 1, the pH values of tannic acid solutions varies between 3.47 and 2.87 in the interval between 1 and 30%.

These values are not far removed from those of the aluminium salts solutions commonly used as anti-perspirants, such as aluminium sulphate, aluminium chloride and aluminium chlorohydroxy complex (Chlorohydrol).

Table 1: Comparison of the pH values, at various concentrations, of tannic acid, aluminium sulphate, aluminium chloride and aluminium chlorohydroxy complex solutions.

| pH value of the | concentration % | | | | |
|---|---|---|---|---|---|
| solutions of | 1 | 5 | 10 | 20 | 30 |
| tannic acid | 3.47 | 2.92 | 2.9 | 2.9 | 2.87 |
| aluminium sulphate | 3.5 | 3.2 | 3.1 | 2.8 | 2.65 |
| aluminium chloride | 3.45 | 2.9 | 2.6 | 2.25 | 1.75 |
| aluminium chlorohydroxy complex | 4.55 | 4.5 | 4.4 | 4.25 | 4.20 |

Obviously, the same astringent effect of tannic acid can be obtained by its derivatives.

To obtain a sustained effect product, tannic acid must be microencapsuled, for example, with an ethylcellulose membrane.

The microencapsulation consists of covering a substance with a polymeric film. The polymer may be soluble in water or in aqueous based liquids such as sweat.

The polymer may also be water insoluble, but permeable, therefore the microencapsulated substance is released for diffusion: namely, water or acqueous vehicles, such as sweat, in our case, penetrate through the membrane, dissolve the active principle, and the solution diffuses on the exterior in times more or less long, which, for a given substance, depends on the percentage of the membrane and the quantity of liquid. In this case, therefore, the membrane exercizes a multiple action:

- extends the dissolution time and, therefore, the deodorant-anti-perspirant action of tannic acid in time;

- regulates the release of tannic acid in relation to the major or minor presence of humidity, which in the case at hand is constituted by perspiration;

- avoids direct and violent contact of elevated quantities of tannic acid with the skin, which permits an increase of an already good tolerability.

The microencapsulation techniques which may be used are various and known to a man skilled in the art.

Amongst those preferred, those specified in U.S. patents Nos. 3,415,758 - 3,155,590 - 3,341,416 - 3,694,372 - 3,531,418 are cited. They are based on the coacervation or phase-separation, which consists of:

a)  dissolving the polymer constituting the membrane in a suitable solvent or microencapsulation vehicle;

b)  suspending in this, the granulate prepared as described hereinbefore, keeping it under stirring with a suitable stirrer;

c)  causing the phase separation of the polymer constituting the membrane with one of the known methods or their combinations. The more common methods are: lowering of temperature, adding a non-solvent to the membrane, adding another polymer more soluble in the vehicle than that constituting the membrane, variations of pH. In this phase, the polymer constituting the membrane deposits itself around the granulate, covering it with a continuous film;

d)  possibly hardening the membrane;

e)  separating the microcapsules from the membrane, for example, by filtering or centrifugation, and drying them.

In the case where the microcapsules are used in a spray-can, it is necressary for them to have particularly small dimensions and, namely, inferior to 300 u and preferably inferior to 150 u.

The percentage of the applied membrane may vary between 2 and 50%, and preferably between 4 and 20%, consequently the tannic acid contents varies between 50 and 98% and preferably between 80 and 96%.

As already said , besides its astringent effect, tannic acid shows a deodorant effect due to its anti-bacterial properties, however in the formulations which are the object of the present invention, it is considered suitable to also add a bactericide in percentages which vary according to the bactericide used, but which are in any case very low and rarely exceeding 1%.

Amongst these, as indicative but not limitative, the following are cited: hexachlorophene(2,2,4'-trichloro-2'-hydroxyphenyl ether), Irgasan DP 300 (Geigy) and the vast range of the ammonium quaternary salts such as alkyldimethyl-benzylammonium chloride, alkyl-trimethylammonium chloride, cetyltrimethylammonium bromide, p-terz-octylphenoxy ethoxyethyl-dimethyl-benzylammonium chloride, and the like.

The addition of a perfume to the deodorant and anti-perspirant formulations has the declared purpose of rendering the formulations pleasant, personalized and basically more acceptable to the user also because of the immediate emphasizing of the olfactory properties.

Besides the use of microencapsulated tannic acid with sustained effect, in the formulations which are object of the present invention, it has been considered suitable to also make use of a microencapsulated perfume, with the purpose of stabilizing the quality of the product and prolonging its effect.

The perfume may be microencapsulated with various methods.

Amongst these, those preferred are those cited in U.S. patents Nos. 2,800,457 - 3,872,024 - 3, 697,437, according to which, a gelatine membrane is placed for coacervation around small droplets of perfume. The gelatine swells in the presence of heat and sweat and, in the case where it is not hardened, it dissolves slowly: also in this case the perfume is released in relation to the temperature and the quantity of humidity, in this case of the perspiration.

The formulation also contains a part of the perfume not microencapsulated, with the aim of obtaining the desired perception immediately upon use.

Besides the tannic acid microcapsules, the perfume microcapsules, the non microencapsulated perfume and the bactericide compound, the formulation is constituted of other components common to deodorant formulations such as, in the case of sprays, softening substances and propellant gases which are accurately chosen so as to guarantee the physical stability of the product and the absence of releasing phenomena of the active principles during storing.

The percentage of tannic acid microcapsules which can be used in the formulations for spraying may vary between 0.1-5% and preferably between 0.2 - 2%.

In the examples which follow, some applicative experiments are illustrated in relation to which, the graphs reported in the figures of the annexed sheets of drawings, have been traced, wherein:

Figure 1 shows the release curve of tannic acid as raw material and when microencapsulated, determined with the apparatus for controlled release in vitro, Diffutest;

Figure 2 shows the same release curve of Figure 1, but determined with the U.S.P.XXI Edition method;

Figure 3 shows the dry volatility curve of the perfume as raw material and when microencapsulated; and

Figure 4 shows the wet volatility curve of the microencapsulated perfume.

The present invention is now illustrated with a few non-limiting examples, which relate to the formulations for spraying, but it is understood that the present invention also relates to less common anti-perspirant formulations, such as sticks, powders, roll-ons, correctly formulated with the aim of avoiding the extraction of the astringent agent and the perfume protected by the microcapsules, before use.

EXAMPLE 1

(a) 158 g. of micronized tannic acid with a grain size inferior to 60 microns, and 13.2 g. of ethylcellulose were added to a 1 l beaker containing 440 g. of cyclohexane.

Under stirring, it was heated to 78°C so as to dissolve the ethylcellulose, and then it was cooled to 25°C. The microcapsules thus obtained were separated for filtering, and dried in an oven with forced air circulation. Once dried, the microcapsules were sieved by means of a sieve with a mesh having an opening of 210 microns.

(b) 20 g. of succinated gelatine were dissolved in a 1 l beaker containing 380 ml of water, heating to 50°C.

100 g. of perfume was poured into the said solution, and it was stirred so as to obtain perfume droplets inferior to 100 microns.

15 ml of a solution of 5% polyphosphate was added, and diluted sodium hydroxide or acetic acid wad added so as to obtain a pH comprised between 3 and 5.

Still stirring, it was slowly cooled to 15°C.

During this phase, the coacervation was effected, namely, the deposition of a gelatine film around the droplets of perfume.

Then 20 g. of highly dispersed silica were added and stirring continued until it was homogeneously distributed.

The microcapsules were separated for filtering. After having dried them in an oven with forced air circulation or in a fluidized bed, they were sieved by means of a sieve having a mesh opening of 210 microns.

(c) The tannic acid and perfume microcapsules, the non microencapsulated perfume and Irgasan DP 300, were mixed in isopropyl myrystate. The mixture obtained was placed in a spray-bomb together with propellant gas. In this, as in other examples, valves with an orifice decidedly superior to the maximum dimensions of the microcapsules (from 3 to 5 times), were used. The content of the prepared cans has the following composition percentage:

| | |
|---|---|
| isopropyl myristate | 5.842 |
| Irgasan DP 300 | 0.085 |
| tannic acid microcapsules | 0.215 |
| perfume microcapsules | 0.720 |
| perfume | 0.138 |
| propellant LPG | 30.000 |
| propellant 11/12 (50:50) | 63.000 |

EXAMPLE 2

(a) In a 17 l container equipped with stirrer and warming jacket,

10.0 kg cyclohexane

0.2 kg ethylcellulose

0.2 kg solid paraffin

2.4 kg ground tannic acid

were placed.

After having heated to 78°C so as to dissolve the ethylcellulose, it was slowly cooled to 28°C.

The microcapsules were separated for filtering and dried in a fluidized bed drier, until elimination of the solvent.

(b) Same process as Example 1.

(c) Spray-bombs were prepared, having the following composition percentage:

| | |
|---|---|
| isopropyl myristate | 3.5 |
| hexachlorophene | 0.2 |
| low viscosity silica oil | 2.1 |
| tannic acid microcapsules | 0.3 |
| perfume microcapsules | 0.7 |
| perfume | 0.2 |
| propellant LPG | 30.0 |
| propellant 11/12 (50:50) | 63.00 |

## EXAMPLE 3

(a) Tannic acid microcapsules were prepared, following the same process of Example 1, but with a membrane ratio:tannic acid of 1:10.

(b) In a beaker equipped with stirrer, 10 g. of gelatine, 10 g. of arabic gum and 600 ml of water were added. It was heated to 50°C and 100 g. of perfume were added.

With vigorous stirring, droplets of perfume inferior to 100 microns were formed. With sodium hydroxide and acetic acid, the pH was altered between 4 and 6, then slow cooling was effected until 15°C. During this phase, the coacervation of the membrane around the droplets of perfume was effected. 80 g. of sodium sulphate anhydride was added, and heated to 25°C. Filtering and drying in fluidized bed was done, adding 20 g. of highly dispersed silica so as to facilitate the operation and hinder the aggregation of the microcapsules. The product obtained was sieved by means of a sieve, the meshes of which have an opening of 210 microns.

(c) Then spray-bombs were prepared, having the following composition percentage:

| | |
|---|---|
| isopropyl myristate | 5.628 |
| Irgasan DP 300 | 0.085 |
| tannic acid microcapsules | 0.429 |
| perfume microcapsules | 0.720 |
| perfume | 0.138 |
| propellant LPG | 30.000 |
| propellant 11/12 (50:50) | 63.000 |

EXAMPLE 4

A marketed product of aluminium salts was used to effect an experiment in practical use on 26 volunteers, being 14 females and 12 males.

After 10 days use of the anti-perspirant formulation, the following questions were asked:

1) Did the anti-perspirant action remain valid during the 12 hours?

-Answer: 45% yes - 55% no

2) Did the perfume remain perceptible during the space of 12 hours?

-Answer: 75% yes - 25% no

3) Were unpleasant effects noted?

-Answer: 18% yes - 82% no

The same experiment was carried out with the samples of Example 3, and the following answers were had:

1) 77% yes - 23% no (P 0.05: "chi squared" test)

2) 75% yes - 25% no

3) 8% yes - 92% no

The said experiment was effected on a relatively small number of persons, but it is sufficient to deduce that the formulation of the present invention shows good characteri-

— 10 —

stics, above all with regard to the regulating effect of perspiration, which resulted as being more prolonged than that of the comparison product in a statistically significant manner.

EXAMPLE 5

The following analytic experiment was set up to verify the effectiveness of the microencapsulation in retarding the dissolution of tannic acid:

-about 1 g. of tannic acid and tannic acid microcapsules, prepared according to description in Example 1, were placed in two distinct 40 ml beakers. 10 ml of a solution of KCl 0.3 M were added to each at 37°C. The beakers were rotated for 60 seconds in a suitable apparatus (Diffutest). The aqueous phase was separated from the undissolved product for filtering and the quantity of tannic acid passed in solution was spectrophotometrically determined. The operation was repeated 8 times.

In the graph of Figure 1, the values found for the free and microencapsulated tannic acid were reported. As can be seen, the dissolution of tannic acid is immediate, whilst that of the microencapsulated acid is more than 8 times slower.

Obviously, the experimental conditions differ decidedly from the real situation, namely, humidity present in the arm-pit in comparison to the 10 ml of aqueous solution used in the experiment in vitro, but permit clear emphasis of the retardant effect of the film applied around tannic acid.

EXAMPLE 6

Another test was effected to show that the membrane of the microcapsules slows the dissolution of tannic acid in water.

For this purpose, an apparatus described in the American Pharmacopoeia (U.S.P. XXI ed., pag. 1224) was used, agitating at a speed of 25 r.p.m.

In 500 ml of distilled water,

-100 mg. of tannic acid raw material

-100 mg. of microcapsules of Example 3, equal therefore to 100 mg. of tannic acid were placed.

The dissolution of the tannic acid was continuously followed, by means of a computerized spectrophotometer, at 274 nm.

The data reported in the graph of Figure 2, emphasize the slowing of the dissolution of tannic acid due to the microencapsulation.

EXAMPLE 7

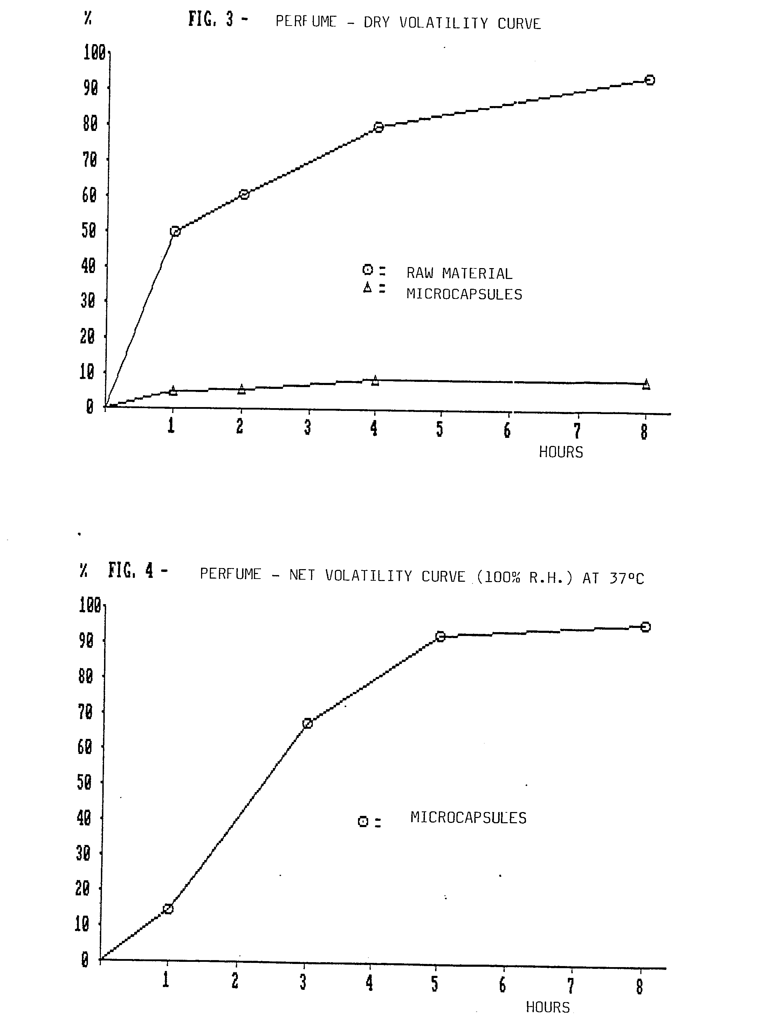

This test as effected to prove that the microcapsules of perfume prepared in Example 1 are stable in the absence of humidity, whilst the perfume not microencapsulated, evaporates rapidly in the same conditions. With this aim, the perfume as such and the same however microencapsulated perfume were placed under vacuum at 37°C for 8 hours. In Figure 3, the values corresponding to loss of weight of the volatile fractions, were reported. The data found, show that the membrane applied in the process of microencapsulation constitutes an effective barrier against evaporation of the perfume in the absence of humidity.

EXAMPLE 8

Instead, in order to study the effect of humidity on the gelatine membrane of the perfume microcapsules, the following test was effected:

Various samples of perfume microcapsules prepared in Example 1, were kept for successive times in a closed environment at a relative humidity of 100% and at 37°C. After each time, the quantity of perfume issuing from the microcapsules under vacuum at 37°C, was measured. It is clear from the results, expressed as percentage of volatile fraction and reported in the Figure 4, that the perfume is gradually released in the presence of humidity. Therefore in the physiologic conditions, the dissolution of the

gelatine membrane, together with the mechanical breakage of the microcapsules due to friction, permits the perfume to be perceived for a long time.

CLAIMS

1. Deodorant and anti-perspirant formulation capable of controlling the perspiration and deodoration levels in time, characterized by the fact that tannic acid microcapsules and/or derivatives thereof are used as an active astringent and deodorant agent.

2. Formulation according to Claim 1, characterized by the fact that it also contains microcapsules of a perfume.

3. Formulation according to Claim 1 and/or 2, characterized by the fact that it also contains an anti-bacterial agent.

4. Formulation according to Claim 3, characterized by the fact that the anti-bacterial agent is preferably chosen among hexachlorophene (2,2,4'-trichloro-2'-hydroxyphenyl ether), Irgasan DP 300 (Geigy) and quarternary ammonium salts such as alkyldimethyl-benzylammonium chloride, alkyl-trimethyl-ammonium chloride, cetyl-trimethyl-ammonium bromide, p-terz-octylphenoxy ethoxyethyl-dimethyl-benzylammonium chloride, and the like.

5. Formulation according to any one of the Claims 1, 2, 3, 4, characterized by the fact that they are prepared in spray form wherein the propellant is constituted of hydrocarbons, alone or in a mixture with fluorinated derivatives.

6. Formulation according to Claim 5, characterized by the fact that in the spraying formulations the percentage of tannic acid microcapsules varies between 0.1 and 5% and preferably between 0.2 and 2%.

7. Formulation according to the preceding Claims, characterized by the fact that it also contains a part of non microencapsulated perfume.

% **FIG. 1 -** TANNIC ACID – "DIFFUTEST" RELEASE CURVE

⊙ = RAW MATERIAL
△ = MICROCAPSULES

NUMBER OF EXTRACTIONS

% **FIG. 2 -** TANNIC ACID – U.S.P./APP.II RELEASE CURVE

⊙ = RAW MATERIAL
△ = MICROCAPSULES

MINUTES

**FIG. 3 -**   PERFUME – DRY VOLATILITY CURVE

% 

⊙ = RAW MATERIAL
△ = MICROCAPSULES

HOURS

**FIG. 4 -**   PERFUME – NET VOLATILITY CURVE (100% R.H.) AT 37°C

%

⊙ = MICROCAPSULES

HOURS